# EUROPEAN PATENT SPECIFICATION

(11) **EP 4 337 108 B1**
(45) Date of publication and mention of the grant of the patent: **24.09.2025**
(21) Application number: 22726230.0
(22) Date of filing: 06.05.2022
(51) Int. Cl.: A61B 17/04, A61B 17/82, A61B 17/06, A61B 17/88, A61L 17/04, A61B 17/84

(54) **SUTURE APPARATUS, SYSTEM, AND METHOD**
NAHTVORRICHTUNG, -SYSTEM UND -VERFAHREN
APPAREIL, SYSTÈME ET PROCÉDÉ DE SUTURE

(30) Priority: 14.05.2021 US 202163188755 P
(43) Date of publication of application: 20.03.2024
(73) Proprietor: A&E Advanced Closure Systems, LLC, Los Angeles CA 90025 (US)
(72) Inventor: WAHL, Joseph, Asbury Park, New Jersey 07712 (US); DEMEDICI, Darren, Middletown, New Jersey 07748 (US)
(74) Representative: Taor, Simon Edward William
(86) International application number: PCT/US2022/028149
(87) International publication number: WO 2022/240692

(56) References cited:
- EP-A1- 3 405 131
- EP-A2- 0 384 819
- US-A- 5 127 413
- US-A- 5 830 234
- US-A1- 2012 109 129

## Description

### CLAIM OF PRIORITY

This application claims the benefit of U.S. Provisional Patent Application Serial No. 63/188,755, filed on May 14, 2021, the benefit of priority of which is claimed hereby.

### FIELD

This disclosure relates to surgical sutures and, more specifically, to surgical sutures for securing bone portions.

### BACKGROUND

A sternotomy may be performed to gain access to the chest cavity of a patient such as during heart surgery and includes cutting the patient's sternum in half. Sternotomy sutures are often used post-sternotomy to lace up or secure the halves of the sternum so that the halves of the sternum will fuse together.

A prior sternotomy suture includes a stainless-steel wire and a needle that is advanced around the cut sternum halves. The needle is cut off from the wire and portions of the wire are twisted together to form a twist-tie. The twisting may be performed by hand or by using a tool to achieve the desired tension in the cable. The twist-tie maintains tension in the wire such that the suture holds the sternum halves tightly together to encourage fusing of the sternum halves.

US5830234A discloses a sternotomy suture that comprises a loop which passes around a split sternum and is twisted until double cabling begins at which point the suture is fastened to itself. The loop preferably comprises a single strand of wire with one end welded to an intermediate point of said strand leaving a portion available for connection to the needle. A curved needle having a cutting tapered point is swaged to the free end of the single strand. Initially the wire is passed around the split sternum and the needle then removed with a pair of diagonal cutting pliers. A twisting tool, having a handle and a hooked portion, passes through the two free loops on opposite sides of the sternum. Twisting force is applied to the tool and transmitted through the hook to the suture causing it to twist and cable. The suture reaches its critical length L_{crit} when the loop is fully cabled but before double cabling begins. At the onset of double cabling the loop reaches its maximum tension T_{crit}. This is the signal to the surgeon to stop turning and to fasten the loop to itself.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 is an elevational view of a suture having a cable extending around halves of a cut sternum and a twist lock formed by the cable that secures the cable about the sternum.
FIG. 2 is a plan view of a suture apparatus used to form the suture of FIG. 1.
FIG. 3 is an enlarged view of the dashed area in FIG. 2 showing woven wires of a cable of the suture apparatus.
FIG. 4 is a cross-sectional view taken across line 4-4 in FIG. 3 showing the wires of the cable in an initial, untensioned configuration near a surface.
FIG. 5 is a view similar to FIG. 4 showing the wires of the cable under tension and flattened out against the surface.
FIG. 6 is an enlarged view of the dashed area of FIG. 1 showing a cut end of the twist lock.
FIG. 7 is a side elevational view of a twister tool that may be connected to the suture apparatus of FIG. 2 after the suture apparatus has been routed around bone portions to twist the cable and form the twist lock.
FIG. 8 is an exploded view of the twister tool of FIG. 7 showing a snap ring, a handle, and a shaft with a hook.
FIG. 9 is a cross-sectional view of the twister tool of FIG. 7 showing the shaft rotatably captured in the handle.
FIG. 10 is a plan view of the snap ring of FIG. 8 showing radial projections of the snap ring that engage a groove of the shaft.
FIGS. 11-17 show a method of forming a figure-eight suture on a cut sternum using the suture apparatus of FIG. 2 and the twister tool of FIG. 7.

### DETAILED DESCRIPTION

In accordance with the invention, a suture apparatus is provided as set out in claim 1. In this manner, the surgical cable may be fixed about the bone portions to stabilize the bone portions by twisting the leading and trailing end portions of the cable together. The twist lock thereby provides a secure locking mechanism to hold the cable about the bone portions that may be formed using the cable itself. Further, the woven shape-retaining wires provide a plurality of load-bearing elements to resist separation of the bone portions.

In one form, the woven shape-retaining wires can bite into one another with twisting of the surgical cable leading and trailing end portions and form a mating fit between the twisted wires which resists relative movement of the wires and loosening of twist lock. Further, the woven shape-retaining wires are configured to plastically deform and inhibit loosening of the woven shape-retaining wires. The deformation of the woven shape-retaining wires caused by twisting the leading and trailing end portions imparts residual stress in the wires which fixes the wires in the twisted configuration and resists loosening of the twist lock. The cable may thereby secure itself in position about the bone portions while keeping the bone portion tight and secured together without a crimp or other locking device.

In another aspect, a suture apparatus system is provided as set out in claim 9. The suture apparatus may thereby be positioned about bone portions and the twister tool utilized to twist the leading and trailing end portions of the surgical cable and cause the woven shape-retaining wires to form a twist-lock of the surgical cable. The twist lock of the surgical cable maintains tension in the surgical cable and secures the surgical cable about the bone portions.

In an example not corresponding to the claimed subject-matter, a method is provided for stabilizing bone portions. The method includes advancing a leader of a suture apparatus about the bone portions. The suture apparatus includes a surgical cable having a plurality of woven shape-retaining wires, a leading end portion connected to the leader, and a trailing end portion. The method includes connecting the leading and trailing end portions of the surgical cable to a twister tool and forming a twist lock of the cable by using the twister tool to twist the leading and trailing end portions of the surgical cable together. The suture apparatus may thereby be advanced about bone portions, the twister tool connected to the suture apparatus, and the twister tool utilized to form the twist lock of the cable. The method provides a straightforward approach to stabilizing bone portions while providing tensile strength via the woven shape-retaining wires.

The above discussion is intended to provide an overview of subject matter of the present patent application. It is not intended to provide an exclusive or exhaustive explanation of the invention. The description below is included to provide further information about the present patent application.

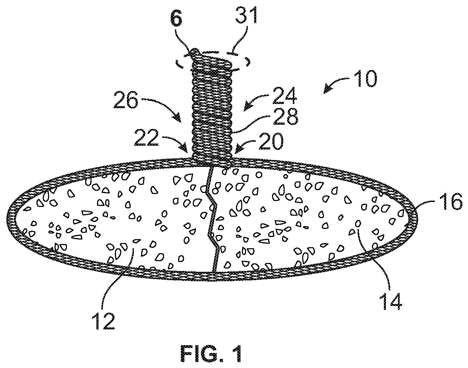

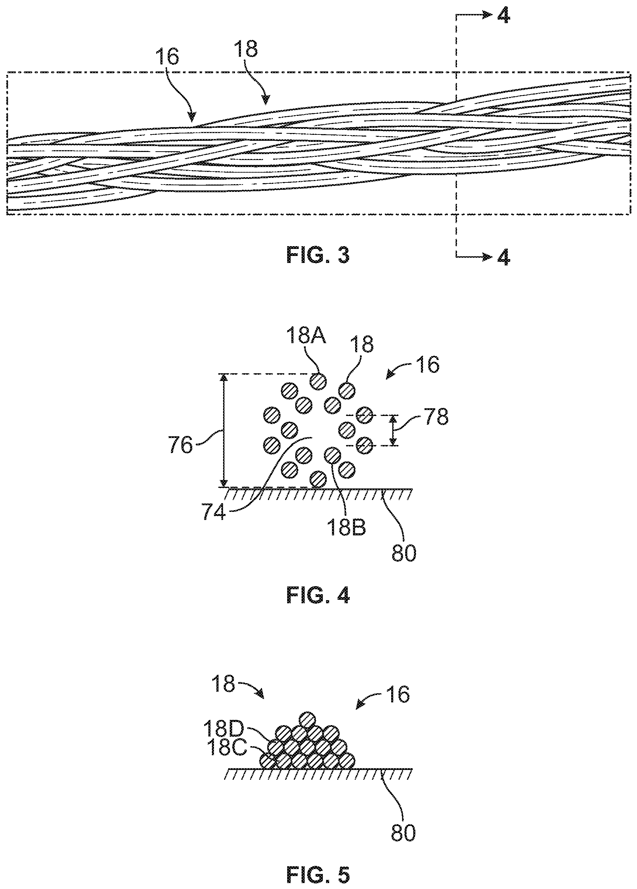

FIG. 1 shows a suture 10 that may be utilized to secure bone portions, such as halves 12, 14 of a cut sternum. The suture 10 includes a cable 16 having a plurality of woven, shape-retaining wires 18 (see FIG. 3). The cable 16 has a leading end portion 20 and a trailing end portion 22 and each of the wires 18 extend the length of the cable 16. The suture 10 includes a twist lock 24 formed by twisting the leading and trailing end portions 20, 22 of the cable 16 together. The twisting of the leading and trailing end portions 20, 22 forms the leading and trailing end portions 20, 22 into intertwined helixes 26 of the leading and trailing end portions 20, 22. The intertwined helixes 26 have a plurality of turns 28 formed by the intertwined leading and trailing end portions 20, 22.

The woven wires 18 of the cable 16 plastically deform with twisting of the leading and trailing end portions 20, 22 into the intertwined helixes 26. As the wires 18 are worked, the wires 18 become more rigid and resist change to their shape. Thus, as the leading and trailing end portions 20, 22 are twisted to form the twist lock 24, each turn 28 of the cable 16 works the lower turns of the cable 16 which further deforms the wires 18 and increases the resistance of the wires 18 to loosening from the tight, intertwined helical shape. Further, the twisting of the leading and trailing end portions 20, 22 also causes the wires 18 to bite or deform into each other with each twist of the twist lock 24 and further resists loosening of the twist lock 24. The wires 18 may bite or deform into each other by, for example, a first wire pressing against a second wire such that an outer surface portion of the first wire deforms an outer surface portion of the second wire. As an example, the wires 18 may each initially have circular cross section perpendicular to the length of the wire. The outer surface portion of the first wire presses against and forms a recess in the outer surface portion of the second wire such that the second wire no longer has a circular cross-section at the outer surface portion. The twisting action urges the outer surface portion of the first wire into the recess of the second wire. Further, the plastic deformation of the wires 18 due to the twisting operation causes the first and second wires to remain in the twisted configuration with the outer surface portion of the first wire engaged with the recess of the outer surface portion of the second wire. The mating or interlocking outer surface portions of the first and second wires 18 further resists relative movement of the first and second wires 18 and further inhibits loosening of the twist lock 24.

As another example, the wires 18 may each initially have circular cross sections and the twisting of the leading and trailing end portions 20, 22 of the cable 16 urges together outer surface portions of first and second wires 18. The outer surface portions of the first and second wires 18 both deform and tightly press against one another. For example, the outer surface portions of the first and second wires 18 may flatten out such that the cross-section of the first and second wires 18 both become non-circular.

In this manner, the deformation of the wires 18 caused by twisting of the leading and trailing end portions 20, 22 rigidly intertwines the wires 18 and forms the twist lock 24. The twist lock 24 fixes the leading and trailing end portions 20, 22 relative to one another and maintains tension in the cable 16, which keeps the sternum halves 12, 14 tightly engaged to facilitate healing of the sternum halves 14, 16.

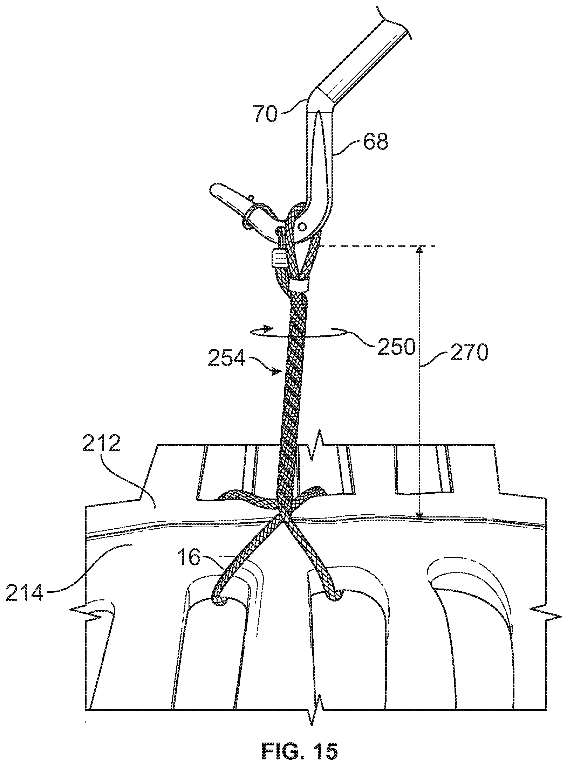

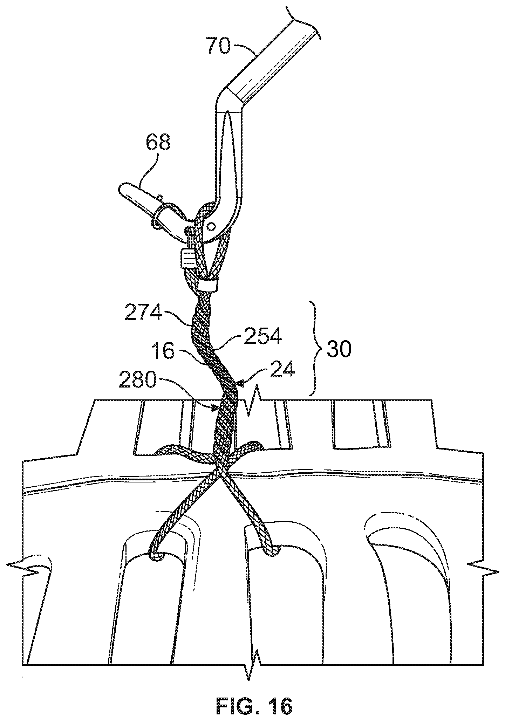

Referring to FIGS. 15-17, to form the twist lock 24, the leading and trailing end portions 20, 22 are twisted using a twister tool 70 to form a cable bundle 254 until double cabling 274 occurs in the cable 16. The double cabling 274 occurs when the cable bundle 254 changes from a single cable bundle into two cable bundles. For example, the double cable 274 may be visually identified when a kinking or a deviation from the straight, column-like arrangement of the cable 16 begins to form as the twister tool 70 twists the leading and trailing end portions 20, 22 of the cable 16. An example of the kinking which forms the double cabling 274 is shown in FIGS. 15 and 16. The double cabling 274 indicates the cable 16 has reached its maximum tension against the sternum halves 12, 14.

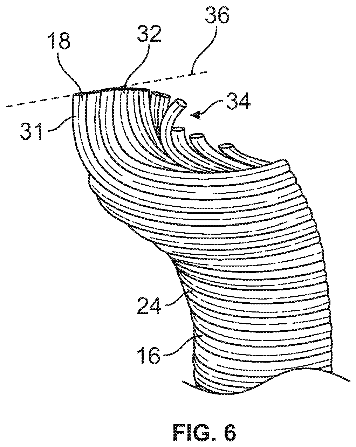

Next, the surgeon may remove an excess portion 30 of the leading and trailing end portions 20, 22 to form a desired length of the twist lock 24. The excess portion 30 is the length of the double cabling 274 beyond the desired length of the twist lock 24. For example, the excess portion 30 may begin a distance in the range of 0.5 inches (1.27 cm) to 2 inches (5.08 cm), such as 1 inch (2.54 cm), away from the sternum halves 212, 214. The removing of the excess portion 30 can include cutting leading and trailing end portions 20, 22, such as using diagonal pliers. The cutting operation shears the wires 18 and forms ends 32 (see FIG. 6) of the wires 18 into an end bundle 34. The shearing of the wires 18 further works the material of the wires 18 and introduces residual stress in the wires 18 so that the ends 32 are rigidly held in the end bundle 34 shape. The tightly bundled shape of the wire ends 32 provides a clean end 31 for the twist lock 24 to minimize irritation to surrounding tissues. The surgeon may then bend the twist lock 24 down against the sternum halves 12, 14 to reduce the profile of the twist lock 24 on the sternum halves 12, 14.

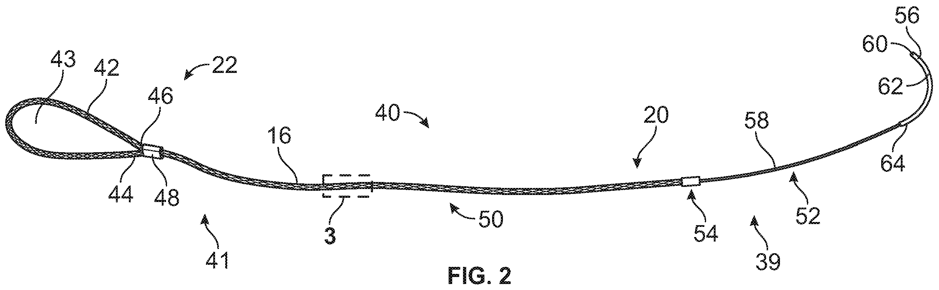

In FIG. 2 a suture apparatus 40 is shown as forming the suture 10. The suture apparatus 40 includes the cable 16 having the wires 18 discussed above. In one embodiment, the suture apparatus 40 includes a single cable 16, although in other embodiments two or more cables 16 may be joined together such as by using a crimp.

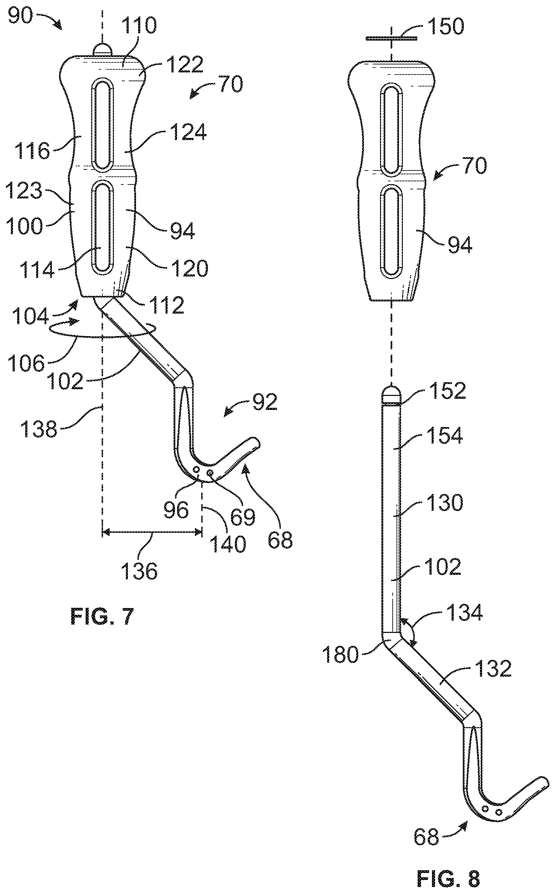

The suture apparatus 40 has a first end portion 39 that includes the leading end portion 20 of the cable 16 and a second end portion 41 that includes the trailing end portion 22. The second end portion 41 includes a loop 42 formed by a portion 44 of the cable 16 secured to another portion 46 by a connector, such as a crimp 48. The loop 42 forms an opening 43 to receive a hook portion 68 (see FIG. 7) of the twister tool 70 when the twister tool 70 is used to form the twist lock 24 in the cable 16.

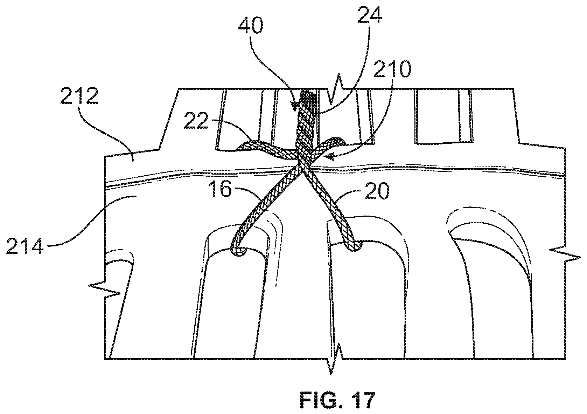

The cable 16 includes an intermediate portion 50 between the trailing end portion 22 and the leading end portion 20. The intermediate portion 50 has a length sized to permit a desired shape of the suture 10 to be formed. For example, the suture 10 in FIG. 1 is a single loop suture with the cable 16 looped once around the sternum halves 12, 14. As another example, the intermediate portion 50 may be longer to permit a surgeon to use the suture apparatus 40 to form either the single loop suture of FIG. 1 or a figure-eight suture as in FIG. 17.

The leading end portion 20 of the cable 16 is connected to a leader 52 by a connector, such as a crimp 54. The crimp 54 may be, for example, a swage fitting. The leader 52 is configured to be advanced into a patient's chest cavity, behind the sternum halves, outward from the chest cavity, and moved away from the sternum to pull the leading end portion 20 of cable 16 into the chest cavity, behind the sternum halves, and outward from the chest cavity. The leader 52 may include a needle, such as a curved sternotomy needle 56, and a lead, such as a monofilament lead wire 58. The needle 56 includes a tip 60, a midsection 62, and a butt section 64 secured to the monofilament lead wire 58, such as by a weld. The monofilament lead wire 58 is connected to the leading end portion 20 of the cable 16 by the crimp 54. Alternative or additional approaches may be used to join the monofilament lead wire 58 to the cable 16, such as a weld or an adhesive.

In one embodiment, the leading end portion 20 of the cable 16 includes a length of the cable 16 extending from the crimp 54 toward the loop 42 a first distance that permits the leading end portion 20 to be twisted with the trailing end portion 22. As some examples in this regard, the leading end portion 20 of the cable 16 may extend approximately a half, third, or quarter of the distance between the crimps 48, 54 depending on a particular application. The trailing end portion 22 of the cable 16 may include the loop 42 and a length of the cable 16 extending from the crimp 48 toward the crimp 54 a second distance that permits the trailing end portion 22 to be twisted with the leading end portion 20. As some examples in this regard, the trailing end portion 22 of the cable 16 may extend a half, a third, or a quarter of the distance between the crimps 48, 54 depending on the application. In some embodiments, the trailing end portion 22 does not include the loop 42.

The wires 18 may be made of a plastic or a metallic material, such as stainless steel or titanium. The wires 18 may be annealed to provide the shape-retentive properties of the wires 18. In one embodiment, the wires 18 are of a super annealed metal material. The term super annealed refers to a material that has been annealed until the material is close to the mechanical limits of the material. For example, the wires 18 may be made of super annealed 316L stainless steel. The wires may be annealed until the wires have mechanical properties that satisfy ASTM F1350, which defines the requirements for surgical grade annealed 316 stainless steel.

Regarding FIG. 4, in one embodiment the wires 18 are monofilaments woven together with the other wires 18 to form the cable 16. For example, the cable 16 may be a 1 x 16 x 0.01 cable, the "1" indicating each wire is woven according to its own pattern, "16" indicating there are 16 total wires in the cable 16, and the "0.01" indicating the diameter of each wire is 0.01 inches (0.0254 cm). In other embodiments, the cable 16 may include multi-filament micro cables each formed of a plurality of wires 18, the multi-filament micro cables being woven together to form the weave of the cable 16. For example, the cable 16 may be a 2 x 8 x 0.01 cable.

In FIG. 4, the wires 18 of the cable 16 include outer wires 18A, inner wires 18B, and an inner space or central void 74. The cable 16 is shown in an initial, untensioned configuration wherein the cable 16 has an effective outer diameter 76 and an effective inner diameter 78. The effective inner diameter 78 may be the distance across the central void 74 the wires 18 naturally assume when the cable 16 is unloaded, such as when the cable 16 is held vertically without tension applied to the cable 16. The wires 18 are loosely woven, meaning that the wires may shift along and about one another as the cable 16 is manipulated. The flexibility of the wires 18 and the ability of the wires 18 to shift along and about one another permits the cable to conform to patient anatomy during positioning and tensioning of the cable 16.

With reference to FIG. 4, the cable 16 is shown near a surface 80, such as the outer surface of a bone. In FIG. 5, the cable 16 has been tensioned which draws the wires 18 against the surface 80. Further, because the wires 18 are loosely woven, the wires may shift laterally and about each other to have a flattened configuration against the surface 80. In particular, the central void 74 has collapsed and the wires 18 are closely adjacent to each other, such as a layer of wires 18C in contact with the surface 80 and one or more layers of wires 18D on the wires 18C. Because the cable 16 may flatten out against the surface 80, the cable 16 may press against the surface 80 over a larger surface area which keeps the cable 16 from biting into the surface 80. Further, the ability of the individual wires 18 to plastically deform and flatten out permits the cable 16 to be wrapped around patient anatomy at sharper angles than normally permitted using a monofilament surgical wire, such as when forming the figure eight suture of FIG. 17.

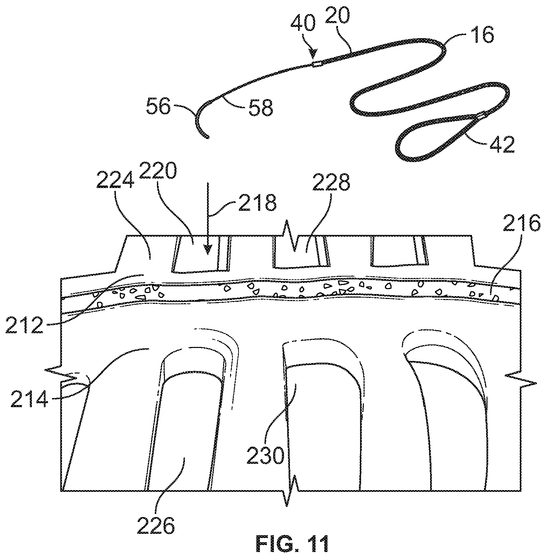

Regarding FIG. 7, the twister tool 70 includes a proximal end portion 90 and a distal end portion 92. The distal end portion 92 is configured to be connected to the leading and trailing end portions 20, 22 of the cable 16 after the suture apparatus 40 has been advanced around bone portions of a patient lying on an operating table. The surgeon grasps the proximal end portion 90 and moves the twister tool 70 away from the patient, which causes the weight of the patient to remove the slack in the cable 16. The surgeon then moves the proximal end portion 90 in a circular or cranking motion to cause the distal end portion 92 to twist the leading and trailing ends 20, 22 of the cable 16 and tension the cable 16 around the bone portions. This process will be described in greater detail with respect to FIGS. 11-17.

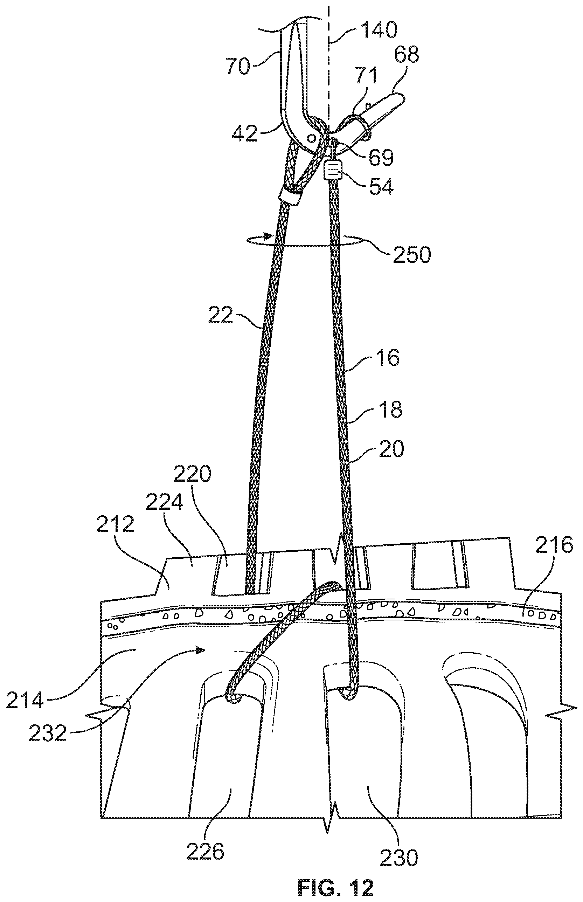

In one embodiment, the proximal end portion 90 of the twister tool 70 includes a handle 94 and the distal end portion 92 includes the hook portion 68 of a support, such as a shaft 102. The leading and trailing end portions 20, 22 of the cable 16 may be connected to the distal end portion 92 of the twister tool 70 by advancing the hook portion 68 through the loop 42 (see FIG. 2) and wrapping the leading end portion 20 and/or monofilament lead wire 58 around the hook portion 68 (see FIG. 12). The twister tool 70 includes a rotary connection 104 between the handle 94 and the shaft 102 that permits the shaft 102 to turn in direction 106 about an axis 138 relative to the handle 94 as the handle 94 is held and moved in a circular motion during twisting of the cable 16, as discussed below.

Regarding FIG. 7, the handle 94 includes a body 100 having a proximal wall 110, a distal wall 112, and through windows or openings 114 formed in a side wall 116 of the handle 94. The handle body 100 has an outer surface 120 and the openings 114 extend radially inward from the outer surface 120 to the shaft 102. To permit the surgeon to maintain a firm grip on the handle 94, the handle 94 has a proximal enlarged portion 122, a narrowed portion 124, and a distal enlarged portion 123. During a twisting operation, the surgeon may grasp the handle 94 with one hand so that the surgeon's index, middle, and thumb fingers are at the narrowed portion 124 and the surgeon's ring and pinky fingers are near the distal enlarged portion 123.

With reference to FIGS. 7 and 8, the shaft 102 includes a straight portion 130, an offset portion 132 extending transversely at an angle 134 relative to the straight portion 130, and the hook portion 68. The angle 134 creates an offset distance 136 between a cable-receiving section 96 of the hook portion 68 and the axis of rotation 138 for the shaft 102. The distance 136 creates a moment arm when the hook portion 68 is secured to the suture apparatus 40 (see FIG. 12). The moment arm generally extends from the connection between the suture apparatus 40 and the hook portion cable-receiving section 96 laterally to the axis 138. With this moment arm, the surgeon may turn the handle 94 in a circular motion in a first direction which causes the handle 94 to travel in circle centered on an axis 140 while the hook portion 68, which is generally held at the axis 140 by the connection to the suture apparatus 40, also turns about the axis 140 in the first direction. The force the surgeon imparts to the handle 94 to move the handle 94 in the cranking or circular motions is transferred to the straight portion 130 of the shaft 102. The offset portion 132 extends between the straight portion 130 and the hook portion 68 of the shaft 102 and operates as a moment arm that applies a torque to the hook portion 68 and leading and trailing ends 20, 22 of the cable 16 connected thereto. The torque applied to the hook portion 68 and the cable leading and trailing end portions 20, 22 connected thereto turns the hook portion 68 and the cable leading and trailing end portions 20, 22 about the axis 140. The turning of the hook portion 68 and the cable leading and trailing end portions 20, 22 about the axis 140 twists the leading and trailing end portions 20, 22 of the cable 16.

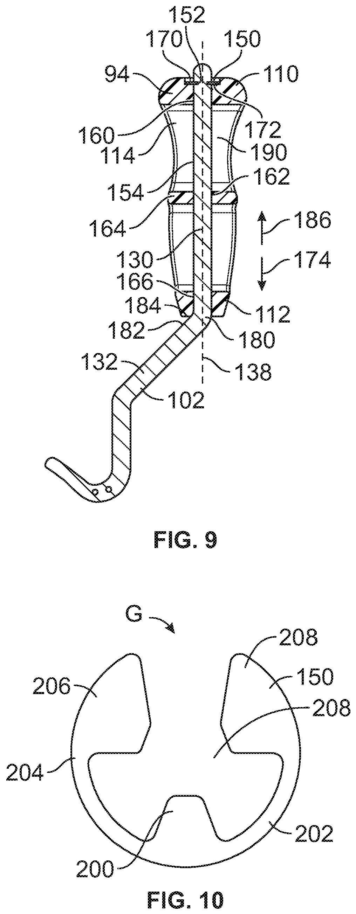

Regarding FIG. 8, the twister tool 70 includes a retainer to releasably secure the handle 94 to the shaft 102. In one embodiment, the retainer includes a snap ring 150 that engages a groove 152 in an outer surface 154 of the straight portion 130. Regarding FIG. 9, the straight portion 130 extends through an opening 160 in the proximal wall 110 of the handle 94, an opening 162 in an intermediate wall 164 of the handle 94, and an opening 166 of the distal wall 112 of the handle 94. The snap ring 150 is received in a recess 170 of the handle proximal wall 110. The snap ring 150 engages the groove 152 of the shaft 102 and is positioned to contact a surface 172 of the proximal wall 110. The contact between the snap ring 150 and surface 172 resists axial movement of the shaft 102 in direction 174 along the axis of rotation 138. The shaft 102 includes an elbow 180 connecting the straight portion 130 to the offset portion 132. The elbow 180 includes an outer surface 182 that contacts a distal surface 184 of the distal wall 112 to inhibit axial movement of the shaft 102 in direction 186 relative to the handle 94. In this manner, the shaft 102 is rotatably captured in the handle 94.

Regarding FIG. 9, the handle 94 facilitates sanitizing of the twister tool 70. More specifically, the twister tool 70 may be sprayed with a solvent to sterilize the twister tool 70. The openings 114 in the handle 94 permit the solvent to travel into the handle 94 and contact the outer surface 154 of the shaft 102 as well as interior surfaces 190 of the handle 94. In some approaches, the twister tool 70 may be disassembled for cleaning. To disassemble the twister tool 70, the snap ring 150 is expanded to disengage one or more projections 200 (see FIG. 10) of the snap ring 150 from the groove 152 of the shaft 102. With the snap ring 150 expanded, the shaft 102 may be removed in direction 174 from the handle 94. The separated handle 94, shaft 102, and snap ring 150 may then be sterilized. In one embodiment, the twister tool 70 is configured to permit some axial movement of the shaft 102 within the handle 94 in directions 186, 174, such as a quarter of an inch (0.635 cm), which makes disassembly and assembly of the twister tool 70 easier.

The handle 94 may have a one-piece unitary construction made of a metallic or plastic material. In other embodiments, the handle 94 may be made of two or more components that are assembled. In one embodiment, the shaft 102 has a unitary, one-piece construction. The shaft 102 may be made of a metallic or plastic material, such as stainless steel.

Regarding FIG. 10, the snap ring 150 has a body 202 that includes the projections 200 extending radially inward from a peripheral portion 204 of the body 202. The snap ring 150 includes ends 206, 208 that are separated by a gap G. The snap ring 150 has an opening 208 that receives a portion of the shaft 102 therein when the snap ring 150 is engaged with the groove 152 of the shaft 102. The snap ring 150 may be made of a resilient material, such as stainless steel.

With reference to FIGS. 11-17, a method is provided for using the suture apparatus 40 and twister tool 70 to form a suture 210 (see FIG. 17) having a figure-eight pattern 232 (see FIG. 12) to secure sternum halves 212, 214. Regarding FIG. 11, the sternum halves 212, 214 are initially separated by a gap 216, such as due to a surgeon cutting the sternum bone.

Initially, the surgeon routes the suture apparatus 40 around the sternum halves 212, 214 to form a figure-eight shape. This routing includes advancing the needle 56 in downward (with reference to FIG. 11) direction 218 through an intercostal space 220 between ribs 224 of the sternum half 212 and into the patient's chest cavity. The needle 56 is then advanced behind the sternum halves 212, 214 within the chest cavity, upward out of the chest cavity through the intercostal space 226 between ribs of sternum half 214, across the upper surface of the sternum halves 212, 214, back into the chest cavity through an intercostal space 228 of the sternum half 212, behind the sternum halves 212, 214 within the chest cavity, and outwardly through an intercostal space 230 of the sternum half 214. As the needle 56 is advanced about the sternum halves 212, 214, the monofilament lead wire 58 draws the leading end portion 20 of the cable 16 along the path created by the needle 56.

Regarding FIG. 12, the needle 56 and leading end portion 20 of the cable 16 have been advanced about the sternum halves 212, 214 to form the figure-eight pattern 232 about the sternum halves 212, 214. The trailing end portion 22 of the cable 16 extends out of the intercostal space 220 and the leading end portion 20 of the cable 16 extends out of the intercostal space 230.

The method includes connecting the leading and trailing end portions 20, 22 of the cable 16 to the hook portion 68 of the twister tool 70. In one embodiment, the trailing end portion 22 is connected to the hook portion 68 of the twister tool 70 by advancing the hook portion 68 through the loop 42 of the cable 16. The leading end portion 20 is connected to the hook portion 68 by first removing the needle 56 from the monofilament lead wire 58, such as by cutting the monofilament lead wire 58, before advancing a portion 71 of the monofilament lead wire 58 through one of the openings 69 of the hook portion 68. The portion 71 of the monofilament lead wire 58 is advanced away from the hook portion 68 to draw the crimp 54 near the hook portion 68. Next, the portion 71 of the monofilament lead wire 58 extending out of the opening 69 of the hook portion 68 is wrapped around the hook portion 68. In another embodiment, a section of the cable leading end portion 20 is advanced through the opening 69 of the hook portion 68 and the section of the cable leading end portion 20 is wrapped around the hook portion 68.

Regarding FIG. 12, with the leading and trailing end portions 20, 22 of the cable connected to the hook portion 68 of the twister tool 70, the handle 94 of the twister tool 70 is cranked or moved in a circular path in direction 250 about the axis 140. The movement of the handle 94 in direction 250 causes the hook portion 68 of the twister tool 70 to start twisting the leading and trailing end portions 20, 22 of the cable 16 together.

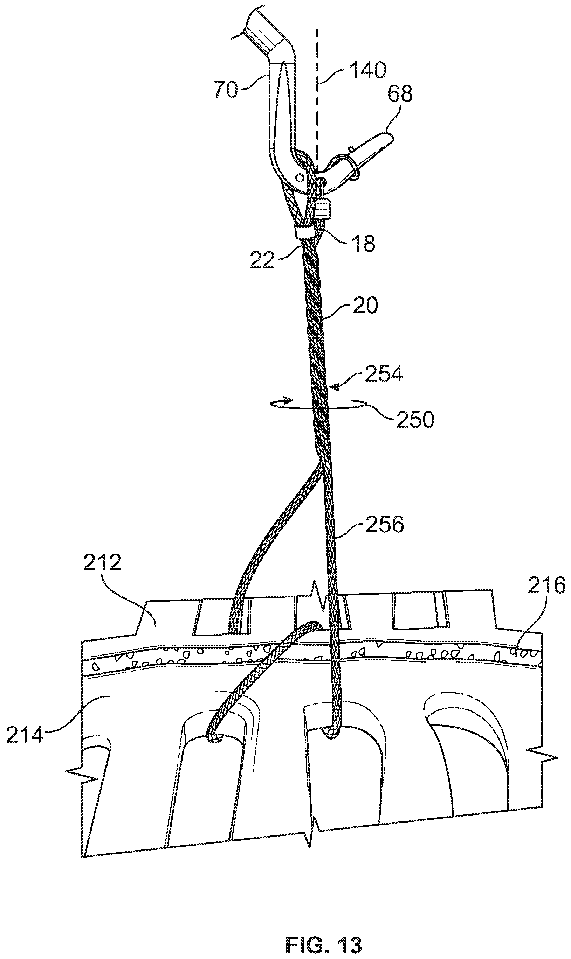

Regarding FIG. 13, the movement of the handle 94 in direction 250 about the axis 140 continues to turn the hook portion 68 in direction 250 and twists the leading and trailing end portions 20, 22 of the wires 18 to form the cable bundle 254 of twisted leading and trailing end portions 20, 22. The cable bundle 254 is initially separated from the sternum halves 212, 214 by straight sections 256 of the cable 16.

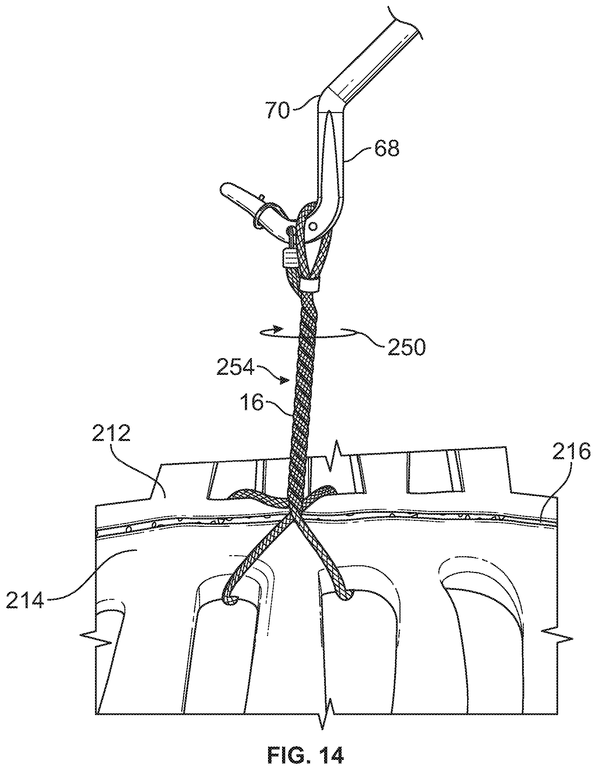

Regarding FIG. 14, the surgeon continues to crank the handle 94 in direction 250 to continue to twist the leading and trailing end portions 20, 22 of the cable 16 together which increases the number of turns of the cable bundle 254 and tightens the turns of the cable bundle 254 until the cable bundle 254 extends all the way from the hook portion 68 to the sternum halves 212, 214. The surgeon may have to rotate the handle 94 several times to twist the cable bundle 254 of FIG. 13 into the form of the cable bundle 254 of FIG. 14.

Comparing FIGS. 14 and 15, the surgeon continues to crank the handle 94 in direction 250 to cause the hook portion 68 to rotate in direction 250. The continued rotation of the hook portion 68 in direction 250 draws more of the cable 16 into the cable bundle 254 and tensions the loops of the cable 16 about the sternum halves 212, 214. The tensioning of the cable 16 urges the sternum halves 212, 214 together and closes the gap 216.

Regarding FIG. 15, the cable bundle 254 has a critical length 270 between the hook portion 68 of the twister tool 70 and the sternum halves 212, 214 wherein the cable bundle 254 extends in a straight column between the hook portion 68 and the sternum halves 212, 214. With the cable bundle 254 having the critical length 270, further cranking of the handle 94 in direction 250 does not substantially increase the compressive force between the sternum halves 212, 214 applied by the cable 16.

Regarding FIG. 16, the method includes further turning or cranking the handle 94 and hook portion 68 in direction 250 until a double cabling 274 occurs in the cable bundle 254. Once the double cabling 274 appears in the cable bundle 254, the surgeon knows that the cable 16 has reached a maximum torque permitted by the cable 16At this juncture, the wires 18 of the cable 16 have been sufficiently worked or deformed by the twisting operation that the wires 18 will rigidly maintain the intertwined helical configuration of the leading and trailing end portions 20, 22. The surgeon may stop rotating the handle 94 about the axis 252 and form a separation 280 in the cable 16, such as by cutting the cable 16. The surgeon then removes the excess portion 30 of the cable 16, which is still connected to the hook portion 68 of the twister tool 70. The excess portion 30 of the cable 16 may be removed from the hook portion 68 and the twister tool 70 sanitized for use in a subsequent operation.

Regarding FIG. 17, the excess portion 30 of the cable 16 has been removed. The twist lock 24 maintains the tension in the cable 16 of the suture apparatus 210 so that the cable 16 may hold the sternum halves 212, 214 tightly engaged together for healing. The twist lock 24 may be pressed down against the sternum halves 212, 214 to reduce the profile of the twist lock 24.

### NOTES

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects. The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims.

## Claims

1. A suture apparatus (40) comprising:
a surgical cable (16) having a plurality of woven shape-retaining wires (18), the surgical cable (16) having a leading end portion (20) and a trailing end portion (22);
a leader (52) connected to the leading end portion (20) of the surgical cable (16), the leader (52) configured to be advanced around bone portions; and
the woven shape-retaining wires (18) of the surgical cable (16) are configured to form a twist lock (24) of the surgical cable (16) by twisting of the surgical cable (16) leading and trailing end portions (20, 22) together,
wherein the woven shape-retaining wires (18) are configured to plastically deform and inhibit loosening of the woven shape-retaining wires (18) by twisting of the surgical cable (16) leading and trailing end portions (20, 22) together to form the twist lock (24).

2. The suture apparatus (40) of claim 1, wherein the woven shape-retaining wires (18) include a super annealed, metallic material.

3. The suture apparatus (40) of any of claims 1-2, wherein the woven shape-retaining wires (18) are each configured to bite into adjacent woven shape-retaining wires (18) and inhibit relative movement between the woven shape-retaining wires (18) by twisting of the surgical cable (16) leading and trailing end portions (20, 22) together to form the twist lock (24).

4. The suture apparatus (40) of any of claims 1-3, wherein the woven shape-retaining wires (18) are configured to form a bunched, free end of woven shape-retaining wires (18) upon cutting of the twist lock (24).

5. The suture apparatus (40) of any of claims 1-4, wherein the trailing end portion (22) of the surgical cable (16) includes a loop (42).

6. The suture apparatus (40) of any of claims 1-5, wherein the leader (52) includes a curved needle (56).

7. The suture apparatus (40) of any of claims 1-6, wherein the surgical cable (16) includes a first end (39) at the leading end portion (20) and a second end (41) at the trailing end portion (22), and wherein the woven shape-retaining wires (18) each extend from the first end (39) to the second end (41).

8. The suture apparatus (40) of any of claims 1-7, wherein the woven shape-retaining wires (18) are configured to shift relative to one another and flatten out against the bone portions as the leading and trailing end portions (20, 22) are twisted to form the twist lock (24).

9. A suture apparatus system comprising:
the suture apparatus (40) of any of claims 1-8; and
a twister tool (70) having a distal connecting portion (92) configured to be connected to the leading and trailing end portion (22) of the surgical cable (16), the twister tool (70) operable to rotate the distal connecting portion (92) to twist the leading and trailing end portions (20, 22) connected thereto and cause the woven shape-retaining wires (18) to form the twist lock (24) of the cable.

10. The suture apparatus system of claim 9, wherein the trailing end portion (22) of the surgical cable (16) includes a loop (42) and the distal connecting portion (92) includes a hook (68) configured to engage the loop (42) of the surgical cable (16) trailing end portion (22).

11. The suture apparatus system of any of claims 9-10, wherein the distal connecting portion (92) of the twister tool (70) includes a hook (68) and at least a portion of the leader (52) is configured to be wrapped around the hook (68).

12. The suture apparatus system of any of claims 9-11, wherein the distal connecting
portion (92) of the twister tool (70) includes an opening (69) and at least one of the leader (52) and the leading end portion (20) of the cable is sized to be advanced at least partially through the opening.

13. The suture apparatus system of any of claims 9-12, wherein the twister tool (70) comprises a shaft (102) including the distal connecting portion (92) and a proximal handle (94) rotatably connected to the shaft (102).

14. The suture apparatus system of claim 13, wherein the shaft (102) extends through at least a portion of the handle, and wherein the handle includes openings that extend inward from an outer surface of the handle to the shaft (102).

15. The suture apparatus system of any of claims 13-14, wherein the shaft (102) is rotatable
relative to the handle about an axis; and
wherein the axis is laterally offset from the distal connecting portion (92) of the twister tool (70).

## Patentansprüche

1. Nahtvorrichtung (40), umfassend:
ein chirurgisches Kabel (16), das eine Vielzahl von gewebten formbeständigen Drähten (18) aufweist, wobei das chirurgische Kabel (16) einen vorderen Endabschnitt (20) und einen hinteren Endabschnitt (22) aufweist;
einen Nahtleiter (52), der mit dem vorderen Endabschnitt (20) des chirurgischen Kabels (16) verbunden ist, wobei der Nahtleiter (52) dazu konfiguriert ist, um Knochenabschnitte herum vorgeschoben zu werden; und
wobei die gewebten formbeständigen Drähte (18) des chirurgischen Kabels (16) dazu konfiguriert sind, eine Verdrehsicherung (24) des chirurgischen Kabels (16) zu bilden, indem der vordere und der hintere Endabschnitt (20, 22) des chirurgischen Kabels (16) miteinander verdreht werden,
wobei die gewebten formbeständigen Drähte (18) dazu konfiguriert sind, sich plastisch zu verformen und ein Lösen der gewebten formbeständigen Drähte (18) zu verhindern, indem der vordere und der hintere Endabschnitt (20, 22) des chirurgischen Kabels (16) miteinander verdreht werden, um die Verdrehsicherung (24) zu bilden.

2. Nahtvorrichtung (40) nach Anspruch 1, wobei die gewebten formbeständigen Drähte (18) ein supergeglühtes, metallisches Material beinhalten.

3. Nahtvorrichtung (40) nach einem der Ansprüche 1 bis 2, wobei die gewebten formbeständigen Drähte (18) jeweils dazu konfiguriert sind, sich in benachbarte gewebte formbeständige Drähte (18) zu beißen und eine Relativbewegung zwischen den gewebten formbeständigen Drähten (18) zu verhindern, indem der vordere und der hintere Endabschnitt (20, 22) des chirurgischen Kabels (16) miteinander verdreht werden, um die Verdrehsicherung (24) zu bilden.

4. Nahtvorrichtung (40) nach einem der Ansprüche 1 bis 3, wobei die gewebten formbeständigen Drähte (18) dazu konfiguriert sind, ein gebündeltes, freies Ende von gewebten formbeständigen Drähten (18) beim Schneiden der Verdrehsicherung (24) zu bilden.

5. Nahtvorrichtung (40) nach einem der Ansprüche 1 bis 4, wobei der hintere Endabschnitt (22) des chirurgischen Kabels (16) eine Schlaufe (42) beinhaltet.

6. Nahtvorrichtung (40) nach einem der Ansprüche 1 bis 5, wobei der Nahtleiter (52) eine gekrümmte Nadel (56) beinhaltet.

7. Nahtvorrichtung (40) nach einem der Ansprüche 1 bis 6, wobei das chirurgische Kabel (16) ein erstes Ende (39) an dem vorderen Endabschnitt (20) und ein zweites Ende (41) an dem hinteren Endabschnitt (22) beinhaltet, und wobei sich die gewebten formbeständigen Drähte (18) jeweils von dem ersten Ende (39) zu dem zweiten Ende (41) erstrecken.

8. Nahtvorrichtung (40) nach einem der Ansprüche 1 bis 7, wobei die gewebten formbeständigen Drähte (18) dazu konfiguriert sind, sich relativ zueinander zu verschieben und sich gegen die Knochenabschnitte abzuflachen, wenn der vordere und der hintere Endabschnitt (20, 22) verdreht werden, um die Verdrehsicherung (24) zu bilden.

9. Nahtvorrichtungssystem, umfassend:
die Nahtvorrichtung (40) nach einem der Ansprüche 1 bis 8; und
ein Verdrehwerkzeug (70), das einen distalen Verbindungsabschnitt (92) aufweist, der dazu konfiguriert ist, mit dem vorderen und dem hinteren Endabschnitt (22) des chirurgischen Kabels (16) verbunden zu werden, wobei das Verdrehwerkzeug (70) dazu betreibbar ist, den distalen Verbindungsabschnitt (92) zu drehen, um den vorderen und den hinteren Endabschnitt (20, 22), die damit verbunden sind, zu verdrehen und zu bewirken, dass die gewebten formbeständigen Drähte (18) die Verdrehsicherung (24) des Kabels bilden.

10. Nahtvorrichtungssystem nach Anspruch 9, wobei der hintere Endabschnitt (22) des chirurgischen Kabels (16) eine Schlaufe (42) beinhaltet und der distale Verbindungsabschnitt (92) einen Haken (68) beinhaltet, der dazu konfiguriert ist, mit der Schlaufe (42) des hinteren Endabschnitts (22) des chirurgischen Kabels (16) in Eingriff zu treten.

11. Nahtvorrichtungssystem nach einem der Ansprüche 9 bis 10, wobei der distale Verbindungsabschnitt (92) des Verdrehwerkzeugs (70) einen Haken (68) beinhaltet und zumindest ein Abschnitt des Nahtleiters (52) dazu konfiguriert ist, um den Haken (68) herum gewickelt zu werden.

12. Nahtvorrichtungssystem nach einem der Ansprüche 9 bis 11, wobei der distale Verbindungsabschnitt (92) des Verdrehwerkzeugs (70) eine Öffnung (69) beinhaltet und zumindest einer von dem Nahtleiter (52) und dem vorderen Endabschnitt (20) des Kabels bemessen ist, um zumindest teilweise durch die Öffnung vorgeschoben zu werden.

13. Nahtvorrichtungssystem nach einem der Ansprüche 9 bis 12, wobei das Verdrehwerkzeug (70) einen Schaft (102), der den distalen Verbindungsabschnitt (92) beinhaltet, und einen proximalen Griff (94), der drehbar mit dem Schaft (102) verbunden ist, umfasst.

14. Nahtvorrichtungssystem nach Anspruch 13, wobei sich der Schaft (102) durch zumindest einen Abschnitt des Griffs erstreckt und wobei der Griff Öffnungen beinhaltet, die sich von einer Außenfläche des Griffs nach innen zu dem Schaft (102) erstrecken.

15. Nahtvorrichtungssystem nach einem der Ansprüche 13 bis 14,
wobei der Schaft (102)
relativ zu dem Griff um eine Achse drehbar ist; und
wobei die Achse seitlich von dem distalen Verbindungsabschnitt (92) des Verdrehwerkzeugs (70) versetzt ist.

## Revendications

1. Appareil de suture (40) comprenant :
un câble chirurgical (16) possédant une pluralité de fils tissés à maintien de forme (18), le câble chirurgical (16) possédant une partie d'extrémité avant (20) et une partie d'extrémité arrière (22) ;
une amorce (52) reliée à la partie d'extrémité avant (20) du câble chirurgical (16), l'amorce (52) étant conçue pour être avancée autour des parties osseuses ; et
les fils tissés à maintien de forme (18) du câble chirurgical (16) sont conçus pour former un verrou de torsion (24) du câble chirurgical (16) en tordant les parties d'extrémité avant et arrière (20, 22) du câble chirurgical (16) ensemble,
lesdits fils tissés à maintien de forme (18) étant conçus pour déformer plastiquement et empêcher le desserrage des fils tissés à maintien de forme (18) en tordant les parties d'extrémité avant et arrière (20, 22) du câble chirurgical (16) ensemble de manière à former le verrou de torsion (24).

2. Appareil de suture (40) selon la revendication 1, lesdits fils tissés à maintien de forme (18) comprenant un matériau métallique super recuit.

3. Appareil de suture (40) selon l'une quelconque des revendications 1 à 2, lesdits fils tissés à maintien de forme (18) étant chacun conçus pour mordre dans des fils tissés à maintien de forme (18) adjacents et empêchant un déplacement relatif entre les fils tissés à maintien de forme (18) en tordant les parties d'extrémité avant et arrière (20, 22) du câble chirurgical (16) ensemble de manière à former le verrou de torsion (24).

4. Appareil de suture (40) selon l'une quelconque des revendications 1 à 3, lesdits fils tissés à maintien de forme (18) étant conçus pour former une extrémité libre groupée de fils tissés à maintien de forme (18) lors de la coupe du verrou de torsion (24).

5. Appareil de suture (40) selon l'une quelconque des revendications 1 à 4, ladite partie d'extrémité arrière (22) du câble chirurgical (16) comprenant une boucle (42).

6. Appareil de suture (40) selon l'une quelconque des revendications 1 à 5, ladite amorce (52) comprenant une aiguille incurvée (56).

7. Appareil de suture (40) selon l'une quelconque des revendications 1 à 6, ledit câble chirurgical (16) comprenant une première extrémité (39) au niveau de la partie d'extrémité avant (20) et une seconde extrémité (41) au niveau de la partie d'extrémité arrière (22), et lesdits fils tissés à maintien de forme (18) s'étendant chacun de la première extrémité (39) à la seconde extrémité (41).

8. Appareil de suture (40) selon l'une quelconque des revendications 1 à 7, lesdits fils tissés à maintien de forme (18) étant conçus pour se déplacer l'un par rapport à l'autre et s'aplatir contre les parties osseuses lorsque les parties d'extrémité avant et arrière (20, 22) sont tordues de manière à former le verrou de torsion (24).

9. Système d'appareil de suture comprenant :
l'appareil de suture (40) selon l'une quelconque des revendications 1 à 8 ; et
un outil de torsion (70) possédant une partie de raccordement distale (92) conçue pour être reliée à la partie d'extrémité avant et arrière (22) du câble chirurgical (16), l'outil de torsion (70) étant utilisable pour faire tourner la partie de raccordement distale (92) de manière à tordre les parties d'extrémité avant et arrière (20, 22) reliées à celle-ci et amener les fils tissés à maintien de forme (18) à former le verrou de torsion (24) du câble.

10. Système d'appareil de suture selon la revendication 9, ladite partie d'extrémité arrière (22) du câble chirurgical (16) comprenant une boucle (42) et ladite partie de raccordement distale (92) comprenant un crochet (68) conçu pour entrer en prise avec la boucle (42) de la partie d'extrémité arrière (22) du câble chirurgical (16).

11. Système d'appareil de suture selon l'une quelconque des revendications 9 à 10, ladite partie de raccordement distale (92) de l'outil de torsion (70) comprenant un crochet (68) et au moins une partie de l'amorce (52) étant conçue pour être enroulée autour du crochet (68).

12. Système d'appareil de suture selon l'une quelconque des revendications 9 à 11, ladite partie de raccordement distale (92) de l'outil de torsion (70) comprenant une ouverture (69) et au moins un élément parmi l'amorce (52) et la partie d'extrémité avant (20) du câble étant dimensionné pour être avancé au moins partiellement à travers l'ouverture.

13. Système d'appareil de suture selon l'une quelconque des revendications 9 à 12, ledit outil de torsion (70) comprenant une tige (102) comprenant la partie de raccordement distale (92) et une poignée proximale (94) reliée de manière rotative à la tige (102).

14. Système d'appareil de suture selon la revendication 13, ladite tige (102) s'étendant à travers au moins une partie de la poignée, et ladite poignée comprenant des ouvertures qui s'étendent vers l'intérieur à partir d'une surface externe de la poignée vers la tige (102).

15. Système d'appareil de suture selon l'une quelconque des revendications 13 à 14,
ladite tige (102) étant rotative par rapport à la poignée autour d'un axe ; et
ledit axe étant décalé latéralement par rapport à la partie de raccordement distale (92) de l'outil de torsion (70).
